Europäisches Patentamt

European Patent Office

Office européen des brevets

(19)

(11) Publication number: 0 348 212
A2

## EUROPEAN PATENT APPLICATION

(12)

(21) Application number: 89306357.8

(22) Date of filing: 23.06.89

(51) Int. Cl.⁴: C 12 Q 1/00
// G01N15/12

(30) Priority: 23.06.88 US 210739

(43) Date of publication of application:
27.12.89 Bulletin 89/52

(84) Designated Contracting States:
AT BE CH DE ES FR GB GR IT LI LU NL SE

(71) Applicant: ATOMIC ENERGY OF CANADA LIMITED
-ENERGIE ATOMIQUE DU CANADA LIMITEE
344 Slater Street
Ottawa Ontario, K1A OS4 (CA)

(72) Inventor: Gentner, Norman E.
R.R. No. 1 Petawawa
Ontario, KOJ 1JO (CA)

Morrison, Donald P.
15 Hammond Court
Deep River Ontario, KOH 1PO (CA)

(74) Representative: Maggs, Michael Norman et al
Kilburn & Strode 30 John Street
GB-London WC1N 2DD (GB)

The title of the invention has been amended (Guidelines for Examination in the EPO, A-III, 7.3).

(54) Method for determining susceptiblity of an organism to a potentially toxic agent.

(57) A method for determining the susceptibility of an organism to a potentially toxic agent comprising: (i) providing a control sample and a treatment sample from cells derived from the organism; (ii) contacting the treatment sample with the potentially toxic agent; (iii) incubating the cells of the treatment and control samples after step (ii), in nutrient medium and then assaying the apparent number of intact cells; and, (iv) determining a measure of susceptibility of the organism to the potentially toxic agent from a comparison of the change in number of apparently intact cells in the control and treatment samples over time. This method can be used to assess the therapeutic regime to give maximum benefit with minimum risk. It can be also used to test applicants for employment where there is an occupational hazard associated with genetoxic agents.

FIG. I

EP 0 348 212 A2

Description

## METHOD FOR DETERMINING SUSCEPTIBILITY OF AN ORGANISM TO A POTENTIALLY TOXIC AGENT

This invention relates to a method for determining the susceptibility of an organism to a potentially toxic agent, a kit for measuring susceptibility of a test subject to a potentially toxic agent, a multicomponent kit for determining the range and extent in susceptibility of individuals in a population of a species of organism to a potentially toxic agent and a method for determining a therapeutic dose or determining or monitoring a treatment regime of an anti-neoplastic agent to an individual organism.

The invention relates to methods for determining the susceptibility of individual members of populations of organisms to genotoxic agents and to define the range and extent of natural variation which exists in human populations with regard to susceptibility to toxic agents.

A wide variety of methods have been developed to assess either the toxicity of agents to standard lines of cells or to assess the susceptibility of particular lines of cells (and by implication, the organism from which they are derived) to particular genotoxic agents.

Kenneth H. Kraemer et al in Mutation Research, 72 (1980) 285-294 detail a method of extrapolating growth curves of surviving cells back to the origin in an attempt to obtain what are assumed to be surviving cell number immediately after treatment (growth curve extrapolation). These surviving cell numbers are taken to indicate the relative toxicity of the agent at the dose administered. The method assumes no response lag.

R.E. Tarone et al in J. Neurologic Sci. (1984), 65, 367-381 describe an assay for detecting hypersensitivity to ionizing radiation in lymphoblastoid cell lines. The method relies upon the ability or lack thereof to take up the vital dye trypan blue. It further employs a single assay of viability ratio at 72 hours.

Philip C. Chen et al in Nature 274, 484-486 (1978) describe the determination of ataxia telangiectasia heterozygotes using colony-forming ability after exposure of cell samples (previously immortalized with Epstein-Barr virus). These workers also studied cell viability determined by trypan blue exclusion.

Hatsumi Nagasawa et al in Cancer Research 47, 398-402 (1987), describe the detection of ataxia telangiectasia heterozygotes using an accumulative labelling index as well as colony forming assay. The accumulative labelling index was determined by the ability of cells, after treatment (with X-rays), to take up radiolabelled thymidine. The results for irradiated cultures were expressed as a ratio of the maximum cumulative labelling index for irradiated as compared with non-irradiated cells.

United States Patent 4,407,942 concerns fluorescent detection of DNA damage caused by, for example, exposure of living cells or organisms to low doses of radiation or chemicals. This patent relates to DNA damage, measured chemically, and thereby the cells' ability to accomplish repair of such damage, rather than to a measure of cell or organism susceptibility.

United States Patent 4,302,535 relates to an assay for determining mutagenesis which comprises exposing cells to a mutagen, incubating the exposed cells for a number of generations to allow full phenotypic expression of resistance to a pyrimidine analogue, culturing such cells in the presence and absence of the analogue and comparing the number of cells for a measure of induced mutagenesis.

United States Patent 4,455,379 relates to a leucocyte adherence inhibition assay which involves fractionating T-cells and culturing these with a radiolabel and subsequently measuring adherence of test and control leucocytes by means of radioassay.

United States Patent 4,582,787 relates to methods of testing patients for predisposition to cancers, especially lung cancer and certain hereditary disorders, by determining the greatest dilution of Kisten murine sarcoma virus which is capable of transforming test cells characterised by a cytological change.

The classical colony forming assay has the drawback that cells, particularly cells of a genotoxin-sensitive type, take time to form colonies and usually then form them slowly. The number of colonies is usually scored at a fixed time after treatment. By no means all cells form colonies and the percentage that do may differ substantially amongst individual donors, thus giving rise to the possibility of selection for a cell type that may not be entirely representative of the donor from which it was derived.

Trypan blue assay has historically suffered from problems of reliability and repeatability, as well as being labour intensive, time consuming and expensive.

Cumulative labelling gives an index which may only be partially indicative of the survival of cells after treatment.

Growth curve extrapolation methods suffer from the problems associated with most extrapolation methods in that (a) they assume all growth delays are uniform, and this may not be the case, particularly when comprising different strains, and (b) artifacts in the results can be magnified on extrapolation leaving the research worker with the dilemma of ignoring a seemingly aberrant point or of including it, each choice comprising an amount of uncertainty that is difficult to quantify.

Another problem which has only partly been addressed to date concerns the effects of dose rate. In theory it may be expected that the effect of the total dose (i.e. a product of dose rate and duration of exposure) would be independent of the dose rate. However, more often than not the effect is related to dose rate, generally becoming more marked as the dose-rate is increased. This means that for comparative purposes dose-rates have to be standardized and to achieve this as many environmental conditions as possible have to be standardized.

Hospital workers working as X-ray technologists, in nuclear medicine, or in a pharmacy dispensing anti-cancer agents are all in positions where screening for carcinogen sensitivity may be warranted. Approximately 10% of all hospital workers in Canada are in such jobs and face an occupational risk higher than normal.

Nuclear workers, especially those in the nuclear power industry may be occupationally exposed to ionizing radiation. Aerospace workers, both airline employees and astronauts, are also likely to be exposed to above-normal levels of radiation.

The United States National Institute for Occupational Safety and Health has designated 49 compounds as being human carcinogens of industrial importance. Many employers, at great expense, screen employees at risk to detect early signs of cancer e.g. leukemic cells. It would be more efficacious and cause less human suffering to detect and protect beforehand those who may be at increased risk of developing cancer on exposure to such agents.

Further, although the environmental causes of malignant neoplasms have received the greatest attention, it is becoming increasingly apparent that predisposing host factors such as genetic variations in response to carcinogen exposure also play a major role, as indicated by the human genetic repertoire of loci whose mutation can significantly increase cancer risk. The study of individual variation in susceptibility to environmental agents is providing new insight into the development of cancer.

These considerations may be relevant to assessing the risk of, for example, ionizing radiation exposure. Should there exist sensitive subgroups at elevated risk, then the average degree of protection implied by regulations directed at radiation protection or occupational health protection will not be achieved for all persons. There has been "increasing recognition that there are human genotypes that confer both increased susceptibility or resistance to DNA damage and increased cancer risk after exposure to carcinogenic agents, including ionizing radiation" and that "if population subsets can be identified as being at substantially greater risk of radiation carcinogenesis, their risk will require separate estimation" (Biological Effects of Ionizing Radiation (BEIR) Report, Washington, DC: National Academy of Sciences, 1980, p. 5). A draft of the document on radiation carcinogenesis in man by the United Nations Scientific Committee on the Effects of Atomic Radiation (UNSCEAR, Vienna: 35th session, 1986, para. 300, repeated as para. 435 in 37th session, 1988) notes that "there may be a non-trivial fraction of the human population which is prone to develop cancer and, as a consequence, may be much more sensitive to radiation and other carcinogenic agents than others", and further, "this may mean that the average dose-response pattern is a relatively poor indicator of individual risk".

There is, therefore, a need for a quick, reliable, and inexpensive test for abnormal sensitivity of individuals to genotoxic agents even though the application of predisposition assays is controversial.

The invention claimed also has great potential utility in cancer treatment. At the moment cancer patients are subjected to particular regimes of treatment with agents which are often genotoxic. In large measure, all patients are subjected to the same treatment procedure for a given tumor type treated with a particular agent. If one was able to assess the individual susceptibility of the patient to a genotoxic agent employed for tumour management purposes one could then adjust the dose up or down to maximize the benefit in attacking the cancer while minimizing the risk to the patient of the toxic effects of the agent.

According to the present invention there is provided a method for determining the susceptibility of individual members of a population of organisms to a potentially toxic agent which comprises: (i) providing a control sample and a treatment sample from cells derived from said organism: (ii) contacting the treatment sample with said potentially toxic agent; (iii) incubating the cells of the treatment and control samples in nutrient medium and then assaying the apparent number of intact cells; and, (iv) determining a measure of susceptibility of said organism to said potentially toxic agent from a comparison of the change in apparent number of intact cells in the control and treatment samples over time.

In a preferred embodiment the method additionally comprises the determining or diagnosing the relative susceptibility of said organism to said potentially toxic agent compared to a reference population of other individual organisms of the same species by (v) comparing the measure of susceptibility of said organism to measures of susceptibility of other individual organisms of the same species in said reference population thereby obtaining a measure of relative susceptibility of said organism to said agent.

The nutrient media which may be used in practice of the invention are determined by the type of cells employed. The concentration and starting number of cells used are not, within wide limits, critical to practice of the invention. Cell types, numbers, concentrations and media employed in the examples are merely illustrative of the method of the invention and, provided that conditions are standardized, are not critical to practice of the invention.

According to another aspect of the present invention there is provided a kit for measuring susceptibility of test subject to a potentially toxic agent which kit comprises at least first and second components which first and second components are in separate containers, said first component comprising at least one, preferably a plurality, reference cell line, each cell line being in a separate container, and said second component comprising at least one incubating medium for grow back of control and treated samples of each cell line.

According to a further aspect of the invention there is also provided a kit for measuring susceptibility of a test subject to a potentially toxic agent to an organism which kit comprises:

(a) sampling means whereby a sample of cells can be withdrawn from said organism;

(b) exposure means whereby a treatment batch of said sample of cells can be exposed to said putative genotoxic agent;

(c) culturing means to culture said treatment batch and said control batch of said sample; and,

(d) measuring means whereby survival slopes of treatment and control batches can be measured and compared to give a measure of susceptibility of said organism to said potentially toxic agent.

Such potentially toxic agents are commonly genotoxic agents, or an agent which can interfere with cell growth or viability.

In particular embodiments of such kits, reference chemicals can be made into parts of the kit to assist in defining normal and susceptible cell lines. Preferably such reference chemicals are from the same broad class of chemicals as the chemical under test so that measures of what is normal and which is susceptible are more likely to be highly correlated between reference and test chemicals.

In other preferred embodiments of such kits at least two reference strains are added to such kits, one of which reference strains is normal and the other one of which is sensitive to the class of toxic agent being tested.

Another aspect of the present invention comprises a multicomponent kit for determining the range and extent in susceptibility of individuals in a population of a species of organism to a potentially toxic agent, which kit comprises a plurality of reference cell strains as components, each component being in a separate container, said reference cell strains having previously been tested for susceptibility to at least one toxic agent, said reference cell strains being selected to vary in susceptibility to said at least one toxic agent among said reference cell strains in a manner which reflects a range of susceptibility of representative individuals sampled from said population of said organism,and said plurality of reference cell strains being large enough to be representative of the range of variation expected in said population. In general, more strains will give one a better indicator of just how suceptible a particular individual is, but, as hinted, these strains need to be chosen with care to be of most use. A "library" of 300 reference strains is more than enough and one can obtain good indications with considerably fewer strains than this (possibly 30).

A further aspect of the invention comprises a method for determining a therapeutic dose or determining or monitoring a treatment regime of an anti-neoplastic agent to be administered to an individual organism suspected of having a neoplasm which method comprises:

(i) providing a control sample and a treatment sample from cells derived from said individual organism;

(ii) contacting the treatment sample with said anti-neoplastic agent;

(iii) incubating the cells of the treatment and control samples, after step (ii), in nutrient medium and then assaying the apparent number of intact cells;

(iv) determining a measure of susceptibility of said individual organism to said anti-neoplastic agent from a comparison of the change in apparent number of intact cells in the control and treatment samples over time;

(v) from said measure of susceptibility, determining a therapeutic dose or determining or monitoring a treatment regime of said anti-neoplastic agent for said individual organism compatible with treatment of a neoplasm in said individual organism and any toxic side effects of said anti-neoplastic agent on said individual organism.

In a preferred embodiment of this aspect of the invention, the therapeutic dose is about the dose which produces the largest difference between a survival rate of neoplasm treatment and a mortality rate from any toxic side effects of the anti-neoplastic agent. This invention is partially useful for determining the best trade-off between the highest therapeutic dose which can be safety administered and the lowest therapeutic dose which is effective for each individual patient. It is a great advance on the empirical methods of determining dose thereto used. These empirical methods give, by trial and error, an average expected effective dose regime based on a test sample from the population rather than a dose regime based on observations made on the individual patient who is to be treated as is the case in this invention.

This invention then is particularly useful in providing a method of determining and/or monitoring a treatment regime for a cancer patient and also in providing a method of optimize radiotherapeutic or chemotherapeutic management of tumours.

From the measure of susceptibility a therapeutic dose or a treatment regime in terms of dose per unit time per unit body weight can be determined or monitored which is compatible with, and a trade-off between, doses and regimes which are effective against a neoplasm and any toxic side-effects of the anti-neoplastic agent on the organism.

The customized therapeutic dose can be determined from previously determined relationships between the susceptibility of representative members of a population of the organism (usually human) and the most effective therapeutic dose which can be applied against such individuals. The material may be, for example, in graphical, tabular or computer memory form. From such a bank of data one can determine a priori a therapeutic dose which all else equal, approximates to an optimun dose for producing maximum therapeutic benefit for minimum risk and harm to the individual being treated. Such an a priori determined dose which accounts for individual susceptibility (or resis tance) can be expected to be much more effective than an empirically determined "best dose" discovered over the years which does not take advantage of an individuals heightened resistance or allow for another individuals increased susceptibility.

In a similar way a treatment regime customized to an individual patient can readily be developed based on the relative susceptibility of the patient determined by the test of the invention. Such a treatment regime can also be monitored during the course of treatment and, as a result of such monitoring, if the optinium treatment dose or regime appears to change then it can be modified accordingly at short notice.

The cells or cell lines may be derived from human sources, especially if one is using the method for occupational screening or cancer therapy. The method may be readily adapted to study other mammals or vertebrates and it is reasonable to extrapolate it to other forms of life that comprise cells and have cellular DNA repair systems for handling the everyday hazards of existence associated with genotoxic agents. The cell lines may be standard laboratory cell lines such as immortalized lymphoblastoid cell lines or peripheral blood lymphocytes isolated from individual donors, especially if the method is used to screen agents for toxicity rather than to screen individuals to determine susceptibility. Lymphocytes which have been stimulated to divide have been found useful in practice of the method.

The organism to which we have given most attention when developing this method is the human. Human cells preferably are used directly for preliminary screening of agents potentially toxic to humans. However, there is no reason to expect that the invention will not work with cells derived from other multicellular organisms or could not be extended to single-celled organisms should the need arise. Examples of animals include most animals of veterinary importance such as cattle, sheep, pigs and goats for screening of "improved" varieties for susceptibility to potentially toxic agents which may normal be present in or may be released into the environment. Cell lines from plants or animals are especially useful when practising the method of the invention as they are inexpensive to use compared to whole organisms and can give good indications therefore of (a) susceptibility of whole organisms to particular potentially toxic agents or (b) just how toxic an agent is to a set of cell lines derived from "normal" (as opposed to susceptible) individuals.

In the method of the invention, when the treatment sample is subjected to step (ii), it is preferable that the control sample undergoes a parallel "sham" control treatment. In one embodiment of the invention, step (iv) comprises comparing the logarithm of the change in number of intact cells in control and treatment samples over time over the time when regrowth is exponential. This can be done, for example, graphically or using a computer to analyze the data.

In the context of the method, acute exposure concerns substantially instantaneous exposure to a relatively high dose of the genotoxic agent in seconds or minutes whereas chronic exposure concerns a rate of delivery of the genotoxic agent which is low, e.g. over a period of hours or days. Acute exposure can be typified as involving a dose which is two or more orders of magnitude greater than a chronic dose. In chronic exposure, the rate of delivery of the genotoxic agent is empirically defined as being relatively slow when compared to the rate of repair of much of the initial damage induced by such a genotoxic agent, so that rate of repair is significant when compared with the rate of damage formation. Under acute exposure the cell-repair systems are, for the duration of the exposure, essentially overwhelmed by the induced damage.

Potentially toxic agents, which include genotoxic agents, can be forms of radiation or chemicals and can be selected from ionizing radiation, e.g., X-rays, or ultra violet radiation, microwaves, or carcinogenic or mutagenic chemicals such as an alkylating agent. Many such potentially toxic agents are selected for study when it is found that they can affect cell growth or viability under certain circumstances. Such potentially toxic agents can then be studied using the method of the invention. Mutagenic and carcinogenic agents are candidates for study using the method. However, agents with no proven toxicity record, but which are routinely encountered as "occupational hazards" can be readily studied using the method to see (a) if there are individuals which are susceptible to an agent which had previously been thought to be totally harmless or, (b) if the substance is, in fact, harmful not just to "susceptibles" but also to "normals". The method can be used to study combinations of agents to see if two separately harmless agents, when combined, affect susceptibility. Such agents may be applied simultaneously or sequentially.

Some specific genotoxic agents which have been studied already include heat, mitomycin C, platinol, 4-nitroquinoline-1-oxide, ultraviolet light (UVA, UVB and UVC wavelengths), ethyl methane sulphonate (EMS) and both low LET and high LET ionizing radiation. Gamma radiation is an agent we have examined particularly closely.

Some chemicals, such as alkylating agents and 4-nitroquinoline-1-oxide, have short half-lives when exposed to cells. Therefore if one wishes to expose cells to chronic doses of such agents one should add the compound either continuously at a slow rate or in the form of small aliquots which are frequently administered.

In the method, determining a measure of the susceptibility of an organism to a potentially toxic agent from a comparison of the apparent change in number of intact cells in control and treatment samples over time can be achieved using a simple Coulter[1] counter. One can also use a multichannel analyser or similar device to score the volume distribution of apparently intact cells. The counts obtained can be graphed or can be stored in a computer. Preferably one compares the logarithm of the change in number of apparent intact cells in control and treatment samples to obtain a grow-back ratio. It is preferred that the results are normalized for intrinsically different growth rates of different strains and this is preferable to using the slope of the regrowth curve of the tested samples alone.

The apparent number of intact cells is scored. This can be accomplished by a number of means such as a Coulter counter or a multi-channel analyser. Many means, such as a multi-channel analyser score everything above a pre-set size as being an intact cell and everything below as being debris. The pre-set size (or channel no.) is set by the operator to obtain the best estimate of the apparent number of cells bearing in mind that small cells will be scored as debris and large pieces of debris will be scored as cells.

Graphed survival slopes (as well as tabulated results) of control and treatment samples reflecting relative rates of grow-back of dividing cells or relative death rates of non-dividing cells can be used as measures of susceptibility or can be used to obtain such a measure: a grow-back ratio or a death ratio.

[1] Trademark

In drawings which illustrate various embodiments of the invention:

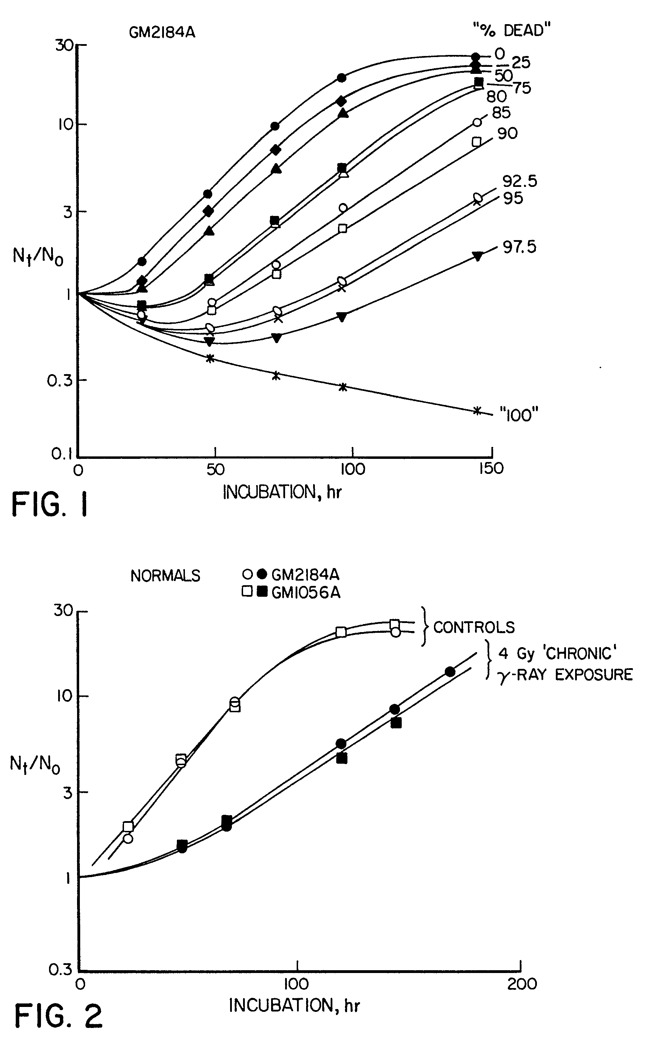

Figure 1 represents grow-back curves for populations of lymphoblastoid cells containing different proportions of live versus dead cells at time zero. An exponential phase culture ($5 \times 10^5$ cells·ml⁻¹) of normal lymphoblastoid cells (GM 2184A) was split into two lots. One was kept as an unirradiated control and the other was exposed to 50 Gy $^{60}$Co γ -rays in oxia; after dilution of each to $10^5$ cells·ml⁻¹, a sample of the former was incubated as "0% dead" (uppermost curve) and a sample of the latter as "100% dead" (lowest curve). The intermediate curves reflect the percentage of 50 Gy-exposed cells compared to unirradiated cells in mixtures of the two lots, all having a total of $10^5$ cells·ml⁻¹ at the start of the incubation period.

Figure 2 shows the grow-back assay for lymphoblastoid lines derived from two normal persons. γ-Exposure conditions were 4 Gy, chronic. For each strain an unirradiated control (open symbols) was run employing a sample of the same lot of cells that were γ-exposed (closed symbols). The Coulter counter cell number ($N_t$) determined at the indicated times are normalized to a starting number of one. The total γ-dose and irradiation method are indicated immediately at the end of the curves to which they apply.

Figure 3 shows the grow-back assay for two A-T homozygous lymphoblastoid lines: all other parameters are as in Figure 2. Myers, D.K. and Gentner, N.E. (1987), Radiat. Environ. Biophys. 26, 263-273 details chronic γ-sensitivity in Roberts syndrome (RS) patients.

Figure 4 shows the grow-back assay for two types of lymphoblastoid strains expected to be moderately radio-sensitive, one (GM 3382) from an A-T heterozygote and one (R20) from a Roberts syndrome patient; all other parameters are as for Figure 2.

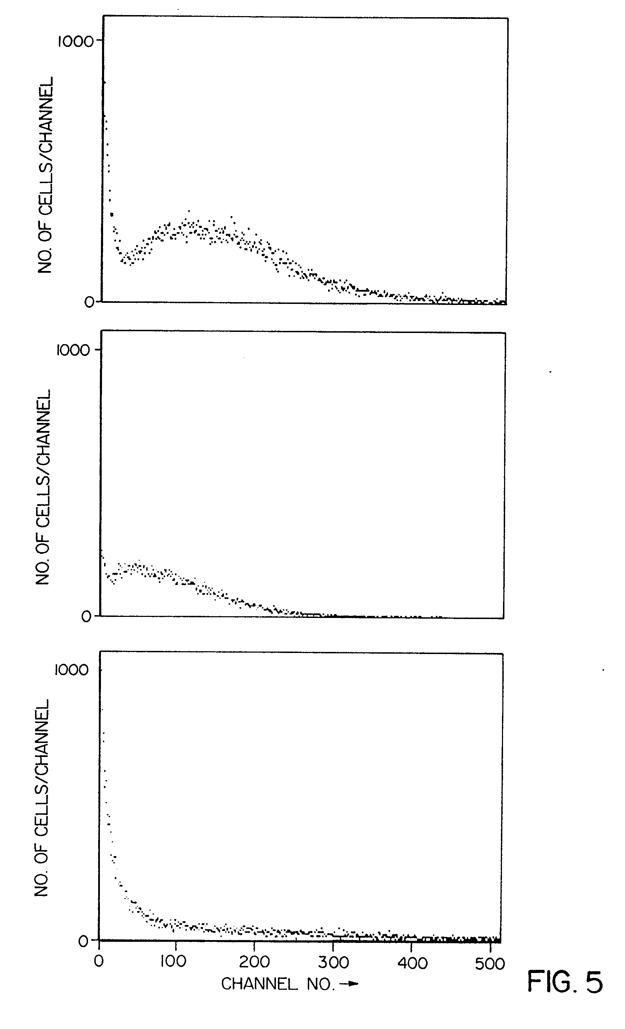

Figure 5 shows multi-channel analyzer profiles of the Coulter counter output; cell number ($10^3$ per channel full scale) versus relative cell volume expressed as channel number (512 total; division marks on the abscissa axis mark 100 channel increments). These cell sizing analyses were conducted after 3 days incubation of normal (upper graph), AT heterozygote (middle graph) and A-T homozygote (lower graph) cell populations exposed to 2.5 Gy (chronic exposure) of $^{60}$Co γ-rays. It is the shape of the size distribution profile that is important here for comparison purposes. The progressive diminution in channel counts in intact cells from channel 40 onwards reflects the greater relative detriment in the intermediate radiation sensitive A-T-heterozygotes and very sensitive A-T-homozygotes occasioned by the radiation exposure.

Figure 6 shows "acute-γ" grow-back ratios at 3.0 Gy and 4.5 Gy for six strains.

Figure 7 shows grow-back ratios obtained using a gamma ray dose of 4 Gy chronic. A "composite normal" and a "composite A-T" grow-back ratio is also plotted.

Figure 8 shows the dose-response curves in the grow-back assay for acute delivery of high LET (14 MeV neutrons) ionizing radiation and for both acute and chronic delivery low LET ($^{60}$Co γ-rays) ionizing radiation. Grow-back ratio is plotted vs. dose. All irradiations were performed in oxia. The GM 2184A acute-γ response is assumed to be consistent with the acute-γ curves for GM 0130B and GM 1056A, as is the case for chronic-γ response.

Figure 9 represents a grow-back assay performed on interleukin-2 stimulated lymphocytes after they were subjected to various amounts of gamma radiation.

Figure 10 shows the distribution of grow-back ratios obtained in the chronic γ-exposure screening assay. Percent of the total number of strains versus GBR (group increments of 0.04). All GBR's less than zero are included in the lowest range. Upper panel: 270 strains from ostensibly normal donors (see Materials and Methods). Middle panel: 11 A-T strains. Lower panel: 27 cancer patient strains. The horizontal bar in the upper panel represents the range of the 25th- to 75th- percentile responses and the dose-modifying factor that is associated with this range.

Figure 11 shows results employing an acute delivery dose test which otherwise parallel those shown in figure 10.

## Description of the Invention

### General Experimental Materials and Methods

#### CELL STRAINS

For the development and validation of the procedures described herein, a selection of lymphoblastoid cell lines (LCLs) was purchased from the NIGMS Human Genetic Cell Repository, Institute for Medical Research, Camden, N.J. In addition to cell lines derived from apparently normal individuals, this selection also included cell lines derived from patients with the inherited disease ataxia-telangiectasia (AT) and from obligate carriers of the syndrome. Patients with this severely debilitating condition, as well as their cells in culture, have been shown to be sensitive to ionizing radiation. While carriers of the condition are apparently normal, careful study has shown them to have some of the features of the patients, but to a lesser extent. Included in these is the increased radiosensitivity of their cultured cells and an elevated lifetime risk of cancer.

Also used in the validation was an LCL (R20) derived from a patient with Roberts Syndrome. This line was obtained from Dr. D.J. Tomkins, Department of Pediatrics, McMaster University, Hamilton, Ontario.

For application of the assay procedure to healthy, ostensibly normal individuals, LCLs for testing were developed by standard methods, see J. Leslie Glick: "Fundamentals of Human Lymphoid Cell Culture", Marcell Dekker, Inc., New York; Basel, 1980, from blood samples donated by employees of Atomic Energy of Canada Research Company at the Chalk River Nuclear Laboratories.

## CULTURE OF LCLs

The cell strains were cultured and maintained using standard techniques (Glick, 1980) (op. cit.). The culture medium used was RPMI 1640 (Gibco Canada Inc.) with the following supplements at the indicated final concentrations: 10% (v/v) fetal calf serum (Flow Laboratories Ltd.), 1mM L-glutamine (Gibco), 100 IU/mL penicillin G and 100μg/mL steptomycin sulfate (Gibco). Culture flasks were incubated at 37°C in a humidified (~-80% relative humidity) atmosphere of 5% $CO_2$/95% air.

## IRRADIATION OF CELLS

Two irradiation protocols were used in defining the "grow-back" procedure. For chronic exposure, cells are grown from an inoculation at approximately 200 000 cells/mL to 1.0-1.3 million cells/mL. At this point the cell cultures were divided into 2 separate lots, control and treatment samples. The cells for treatment, in the medium in which they were grown, were subsequently exposed in a 37°C, 5% $CO_2$/95% air incubator to $^{60}Co$ γ-rays from a Gamma-beam 150 beam port irradiator (Atomic Energy of Canada Ltd.) at a dose rate of 0.0035 Gy/min. The control sample was maintained for a time equal to the exposure time of the treatment sample in a separate, unexposed incubator but with the same temperature and gassing conditions.

For acute exposures, the cell cultures were grown to about 500,000 cells/mL and split into control and treatment samples. The exposure of the treatment sample to $^{60}Co$ γ-rays was performed in air at ambient temperature (20-22°C) in a Gamma-cell 200* (Atomic Energy of Canada Ltd) at a dose rate of 3 Gy/min. For such short exposures no special handling of the control sample was necessary.

## POST-TREATMENT HANDLING AND MONITORING OF GROWTH

On completion of the irradiation procedures a sample of each culture was removed, diluted in Isoton[2] (Coulter Electronics) and the number of cells measured using a Coulter Particle Counter (Coulter Electronics). Each of the control and treatment cultures were subsequently diluted in fresh medium to ~ 100 000 cells/mL and these cell numbers checked as above. The number obtained from this last count ($N_o$) were used in the normalization of subsequent counts ($N_t$). These cultures were incubated under the standard conditions described earlier. The sampling, dilution and counting of the cultures were repeated at regular intervals (usually daily) for up to 7 days to establish the regrowth rate of the control and treated cultures. The growth response was plotted as $N_t/N_o$ on a logarithmic scale vs. incubation time.

## DETERMINATION OF THE GROWBACK RATIO

Irradiated (treated) cells were found to "grow-back" at a rate slower than unirradiated (control)cells. Exponential growth was usually re-established within 20-50 hours of post-treatment incubation. This was dependent on the magnitude of the dose and the inherent sensitivity of the strain.

The slope of the growth curve once exponential growth is re-established was used in the determination of the "growback ratio" (GBR). The GBR is defined as the ratio of the slope of the initial exponential regrowth phase for the treated cell population to that of the unirradiated, control cells of the same strain in the same experiment.

Cell types which we have studied and which can be used in practice of the invention include lymphoblastoid cells which have been immortalized, lymphocytes which have been stimulated to divide (e.g., by a mitogen such as phytohaemagglutinin and a growth factor such as interleukin-2), and fibroblasts. (Other cell types which can be used in practice of the invention include those cells which have not been stimulated to divide, or do not divide, if one wishes to study relative mortality rather than a grow-back ratio.) So long as the cell type can be incubated and can be relied upon to be an indicator of susceptibility of the individual from which is was derived, it can be used in the method.

The nutrient medium required to maintain the cells and, if required, to allow them to multiply, should be a nutrient medium compatible with those cells and is preferably a standard, readily-obtainable medium. Any interaction between the medium and the toxic agent can be accounted for by suitable control experiments.

The cell densities employed typically are those indicated above and the sizes of the samples employed is a matter of convenience, determined in large part by the counting means which one applies.

The dose ranges, particularly when studying a novel (possibly toxic) agent or a previously unstudied cell line, are determined, in the first stage, empirically. Once a particular range of doses has been found where differences in cell number have been demonstrated then one can "home in" on a dose range which best demonstrates the effect. Of course, if the agent is not toxic or the cells not susceptible then no effect will be found. In the case of gamma rays doses in the range of 1.5 to 4.5 Gy were found most useful.

In general, we have found that the effects can be demonstrated upon incubating for up to about 150 hours after exposure. No longer time period is usually needed. In cases where the cells divide rapidly (e.g. microorganisms at high temperatures) the duration of incubation can be correspondingly reduced.

[2] Trademark

MITOMYCIN (MMC) TREATMENT OF CELLS

The cell cultures were grown to about $5 * 10^5$ cells/mL. Two samples of approximately 2 million cells each were dispensed to separate centrifuge tubes and the cells pelleted at about 180 g for 10min. The supernatants were discarded and the cell aliquots resuspended in supplemented RPMI 1640 with or without MMC. The concentration of MMC used for these chronic treatments was 30 ng/mL. The resuspension volume was such that the starting level was about $1 * 10^5$ cells/mL.

In the mitomycin C experiments a "slope ratio" based on the difference in cell numbers between samplings at fixed times (24 and 72 hours) post-MMC introduction was used. This represents essentially the same analysis method as applied to the case of ionizing radiation since the time points used here fall on the exponential portion of the regrowth curves.

Example 1

Mixing experiments to demonstrate factors affecting grow-back.

An exponential phase (i.e. actively growing) culture of a normal lymphoblastoid cell line (LCL) (GM2184A) was split into two aliquots. One was kept as an unirradiated sample and the other was exposed to a very high dose (50Gy) of $^{60}$Co $\gamma$-rays. On completion of the exposure, each of the samples was diluted to ~ 100,000 cells/mL. Mixtures of these dilutions were then used to produce a series of cultures with a range of percentages of live (unirradiated) and "dead" (heavily irradiated) cells, but all at a starting level ~ 100,000 cells/mL. These cultures were then sampled to obtain an initial count ($N_o$) and incubated under our standard growth conditions. Over a period of about a week samples were removed to obtain updated counts ($N_t$) of the cells in the cultures. Figure 1 shows the results of plotting the ratio $N_t/N_o$ against incubation time for 11 cultures, each with a different proportion of "dead" cells. In the 100% "dead" culture (a 50Gy exposure in air may result in survival of the order of less than 1 cell in $10^{10}$) the decline in numbers appears to be essentially exponential up to at least 6 days. The balance between this progressive loss of killed cells and the exponential growth of survivors would seem to account in part for the substantial period of diminished but exponential "grow-back" of an irradiated population.

This example illustrates that part of the apparent delay in the resumption of exponential growth of irradiated cells and the altered slope (that is the basis of the determination of the growback ratio) are the net result of two opposing factors: increasing cell number due to division of those cells that survived the $\gamma$-exposure and decreasing cell number due to the loss of killed cells, presumably by lysis. It should be noted that the live cells had not been irradiated in this experiment.

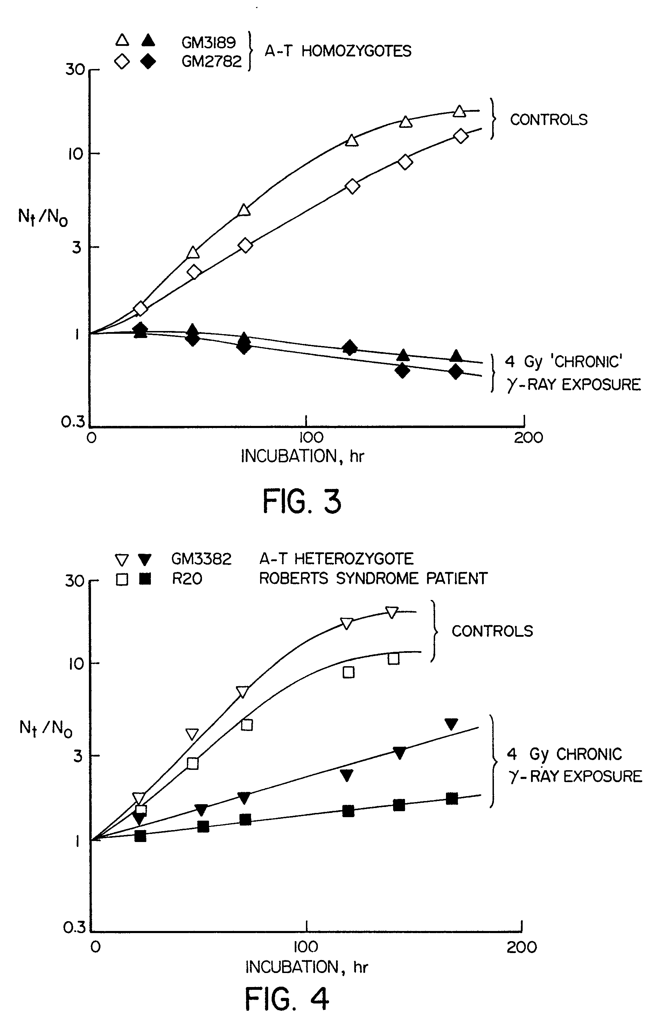

The "grow-back response" after 4 Gy chronic $\gamma$-exposure of two normal lymphoblastoid strains (figure 2), two A-T homozygous strains (figure 3), and two "moderately radio-sensitive" strains of the sort one should be able to detect in a population screening assay (figure 4) (an A-T heterozygous strain and a Roberts syndrome strain) was studied. Clearly, from figures 2-4 the latter two types of lymphoblastoid strains, expected to be radio-sensitive from studies on the clonogenic survival of cultured fibroblasts after exposure to chronic $\gamma$-radiation, are distinguishable from the normal strains. One type of data analysis that can be applied to this grow-back assay is shown in Table I; the distinction between normal strains and "moderately radio-sensitive" strains by this assay is more pronounced than that obtained by Tarone et al. (1984) (op. cit.) using lymphoblastoid cells, acute X-irradiation, and a dye-exclusion assay for viability. Moreover, the expedient of being able to utilize a simple Coulter counter cell tally enormously reduces the time and effort required.

When an acute $\gamma$-exposure regime is employed with the grow-back assay and lymphoblastoid cells, a resolution in radio-response between normal and A-T heterozygous strains was not achieved at any of a variety of doses.

Example 2 Multichannel analyser (MCA) techniques for scoring cell numbers in "grow-back" and related methods

Cell volume measurements, obtained using a Coulter counter and multichannel analyzer (MCA), can be used to distinguish live and dead lymphoblastoid cells in an irradiated population. This was based on procedures developed by Attallah and Johnson: J. Immunol. Methods 41 155-162 (1981) with other types of cells in culture. This method was studied in some detail and found to offer no appreciable advantage over the simpler Coulter counter cell number tally. This example briefly documents these observations.

Attallah and Johnson (1981) claimed that their procedure gave discernible MCA peaks for live and dead cells. Under the conditions employed here, at least, this is not the case for irradiated lymphoblastoid cell populations. The three plots in Figure 5 can be used conveniently and briefly to illustrate several points in relation to the decision to rely on a Coulter Counter. Shown in Figure 5 are three MCA profiles, for a normal strain (top figure), an A-T heterozygote strain (middle figure) and an A-T strain, at 3 days incubation following 2.5 Gy chronic $\gamma$-exposure. In the upper figure, a relatively sharp initial peak is seen at small particle sizes (essentially debris); this accumulates with cell growth, and in an exponentially growing unirradiated cell culture typically represents 10% of the total number of counts. To the right, starting about at channel number 40, a peak representing "viable" lymphoblastoid cells is seen (for unirradiated cells, it would be somewhat sharper and narrower than seen here, with a peak about channel 80). The MCA gives the distribution of number of particles versus particle size (volume expressed as channel no.), and the relevant parameter derived, for the purpose originally stated, is the number of cells contained within the volume distribution representative of viable cells. When a lethal $\gamma$-exposure was employed, it was found that there was no "clean" transition from the "viable" peak to the debris region indicative of cell death; rather, the viable peak collapses toward the left, and no evident division line between viable and non-viable cells is available. The net effect is that the result at higher doses for a normal strain is the same as shown here for the more radio-sensitive A-T strain exposed to a lower dose (bottom graph, Figure 5): a continuous curve, from the debris region out to that occupied by viable cells in the absence of radiation. In such a situation, it is unclear what the appropriate choice would be for the division between viable and non-viable cells, and the viable cell number obtained is exquisitely sensitive to the placement of this division. In the middle graph of Figure 5, representing an A-T heterozygote strain, an intermediate effect is evident, and a choice of an appropriate division point is still possible, as for the normal strain.

At relatively high ionizing radiation doses, where little or no out-growth occurs, this effectively precluded utilization of this procedure to investigate factors related to possible differences in death rate between strains. These investigations did, however, suggest that cells lyse relatively quickly when they become "dead".

The MCA viable cell count can still be used effectively, at lower radiation exposures and effect levels such as for the upper 2 cases in Figure 5, to determine "grow-back" (net increase in viable cell number), but it offers no appreciable advantage under such circumstances to justify its additional complication over a simple Coulter counter tally number. A comparison was made and plotted on the grow-back assay of the invention to determine relative radiosensitivity of $N_t/N_o$ determinations versus time of incubation from the cell number in the "viable cell peak" as determined by the multichannel analyzer coupled to a Coulter particle counter output and from the direct Coulter counter cell tally. In unirradiated cells or at lower $\gamma$-ray exposures, the MCA line was slightly above the Coulter counter line; at higher exposures (those where no grow-back is evident in the time period shown), these positions were reversed. Thus, although the MCA method does offer a limited degree of improved discrimination, over the Coulter counter method, between one strain that does grow-back and another that does not at a given dose (e.g. between a normal strain and one from an A-T patient, as in Figure 2 versus Figure 3 or in the uppermost versus lowermost cases in Figure 5), these differences are so compelling anyway that application of the MCA method to the determination of viable cell number is not warranted. The same conclusion holds for detection of moderately radio-sensitive phenotypes at doses where they also "grow-back" but at a lower rate than normal strains; a grow-back ratio determined from MCA analyses is going to offer resolution similar to that for the ratio derived from the Coulter counter.

The cell volume distributions measured by the MCA have, however, proven extremely useful in evaluating the relative "health" of lymphoblastoid cell cultures for which radio-response is to be assessed. A broadening and shift to the right of a strain's MCA profile, we have found, is the earliest indication of a growth perturbation.

Further evidence suggesting that dead cells lyse, and therefore supporting utilization of the direct Coulter counter tally as being related to viable cells, came from studies utilizing the vital dye trypan blue. Laboratories utilizing this assay for studying the cytotoxic effects of ionizing radiation seem to have reported only the non-trypan blue-staining (i.e. viable) values (Chen et al. 1978; Tarone et al. 1984); data on "dead" cell numbers, (i.e. non-viable, trypan blue-staining cells) are curiously absent. In the early portion of the present study, the trypan blue (TB) procedure was investigated in parallel with the methods being developed; special care was taken in our studies to enumerate the frequency of both TB-staining and non-staining cells.

Using the viable cell number (non-TB-staining), facile discrimination between a normal and an A-T strain was possible following acute $\gamma$-exposure. A non-viable cell number determination is ineffectual in this regard: little or no difference, for example, was usually evident between irradiated and unirradiated cells for a given culture; the accumulation of TB-staining cells seemed to be largely a function of time in culture. In other experiments, utilizing highly lethal exposures such that substantial decreases in non-staining cells occurred, the increases in TB-staining cells have been observed to fall far short of accounting for these differences; this was taken as supporting a conclusion that cells which cease to exclude this vital dye rapidly break up or disintegrate.

Overview of the grow-back assay

We observed that lymphoblastoid cell death, as determined by diminished counts in a Coulter particle analyzer in cultures exposed to relatively high $^{60}$Co $\gamma$-ray doses, appears to reach a limiting rate. It was found that essentially no difference in death rate was evident between cultures exposed to 12 Gy or to 50 Gy. If a cell is destined to die anyway, it appears not to die faster (by the criterion employed) if killed several-times over. From consideration of the doses required to affect reproductive survival and from "reconstruction" experiments such as depicted in Figure 1, this limiting rate seems to be evident, for practical purposes, at exposures where > 99% of the initial number of cells are inactivated. This reduction in apparent cell number is believed to be attributable to lysis (as discussed earlier) and occurs with approximately exponential kinetics. Normal cell reproduction also occurs exponentially. In an irradiated cell population where intermediate numbers of cells survive, there exists a substantial period of diminished (compared to the rate in an unirradiated control population) but exponential "grow-back" in the irradiated population (see Figures 2 and 4; curves for 4 Gy chronic $\gamma$-exposure). This would seem to be in large part the net result of two opposing factors: increasing cell numbers due to division, after a lag period in exposed cells, of those that survived the initial exposure and decreasing cell number due to loss of dead cells, presumably by lysis, from the Coulter counter tally. But it is not so simple as this. If the observed response was due to just the aforementioned factors, the grow-back curve would be expected to become progressively steeper with continued incubation, as a larger proportion of live cells was formed in the population; this is evident to some degree in reconstruction experiments we have performed, of the type shown in Figure 1, where unirradiated and lethally-irradiated cells are mixed in different proportions. What we have also found is that dead cells (or cells destined to die) "use up" the growth medium in an as-yet incompletely understood manner, and diminish the capacity of the nutritive medium to support cell growth (in terms of the final cell number and, to a lesser extent, in growth rate itself); this effect seems to be dose-dependent (data not shown). Moreover, an initial division delay engendered by the radiation exposure occurs (observed microscopically in radiated cells suspended in gel support medium), so mixing experiments of the type depicted in Figure 1 may not be suitable for use in calibrating presumed initial survival levels.

Acute γ-exposure suffices to detect radio-sensitivity in A-T homozygous but not heterozygous strains

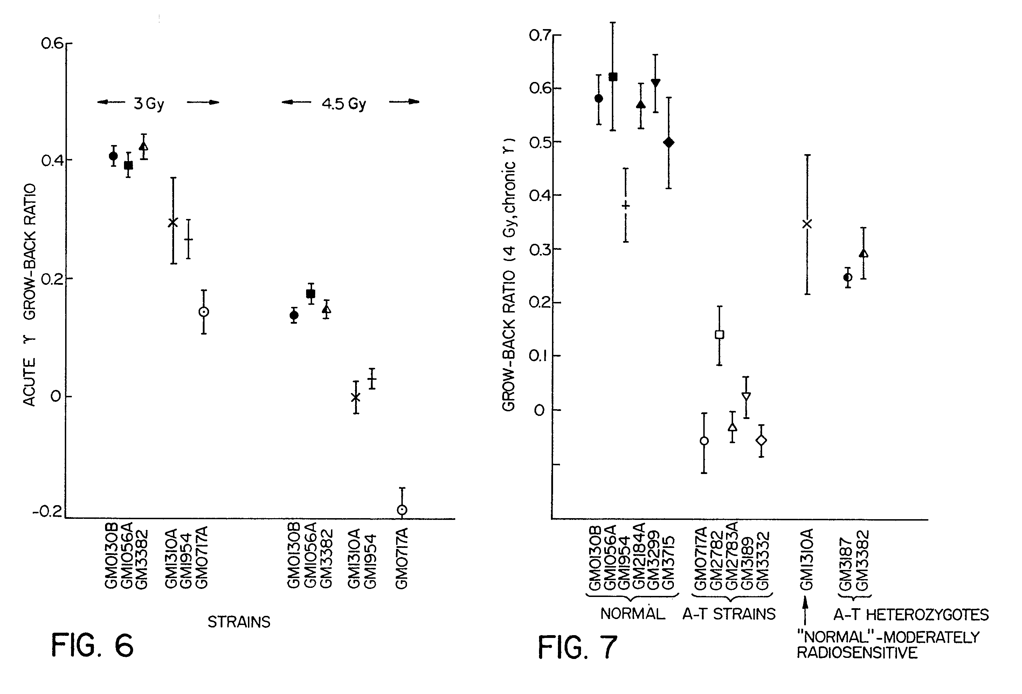

Acute -exposure in oxia was inadequate to elicit a hypersensitive radio-response in lymphoblastoid cells from an A-T heterozygote strain (GM3382; Fig. 6). This is also the case for cultured dermal fibroblasts from such donors (Paterson et al., 1985c (op. cit.); Figure 1 therein); in oxia, protracted γ-dose delivery seems mandatory for detection of this particular moderately radio-sensitive subgroup, both by the grow-back assay (cf. Figures 2 and 4; Table I) and by the survival of colony-forming ability assay employing fibroblasts (Paterson et al., 1985c (op. cit.), Figures 3 and 4 therein).

Normal vs. A-T strains at various γ-ray doses delivered chronically

The discrimination between the radio-sensitivity of normal and A-T strains on chronic γ-exposure was apparent at 4 Gy in Figures 2 and 3. As just described for acute exposure, a range of chronic γ-ray doses has also been studied as regards their utility in defining a difference in radio-sensitivity between normal and A-T strains. A clear distinction was evident between the grow-back response of the normal strain, GM 1056A, and those of the two A-T strains, GM 0717A and GM 3189, at any of a number of the chronic γ-ray doses tested, i.e. 2 Gy, 4 Gy, and 6 Gy. Moreover, the responses of the two A-T strains were remarkably similar at all doses, and replicate determinations showed good reproducibility.

Testing the A-T strain GM 3189 against the apparently normal GM 2184A strain for grow-back response after treatment with high LET ionizing radiation (14 MeV neutrons) showed the A-T cell line to have a hypersensitive grow-back response when compared with the control.

Example 3 Effect of dose rate upon ability to discriminate with the grow-back assay

The results presented in Figures 2-4 demonstrate that the use of reduced dose rate (i.e. chronic exposure) makes possible the identification of strains of normal, intermediate and very high sensitivity. A set of cultures comprising 2 established from apparently normal individuals (GM 2184A and GM 1056A), 2 from AT patient (GM 3189 and GM 2782), one from an AT carrier (GM 3382) and one from a Roberts syndrome patient (R20), were prepared for treatment using the chronic irradiation protocol as described in Experimental Materials and Methods. The treated samples of each culture were exposed chronically to 4 Gy $^{60}$ Co γ-rays. The post-treatment handling and counting were as described earlier. Clearly the lines of which intermediate sensitivity is expected (GM 3382 and R20) based on clonogenic survival of cultured fibroblasts from such individuals are differentiable from both the normal and hypersensitive lines.

Example 4 Comparison of chronic vs. acute γ-exposures in the grow-back assay (Fig. 6 and Fig. 7)

The data obtained in the grow-back screening assay allow some informative comparisons to be made among strains with respect to their response to chronic versus acute γ-exposure.

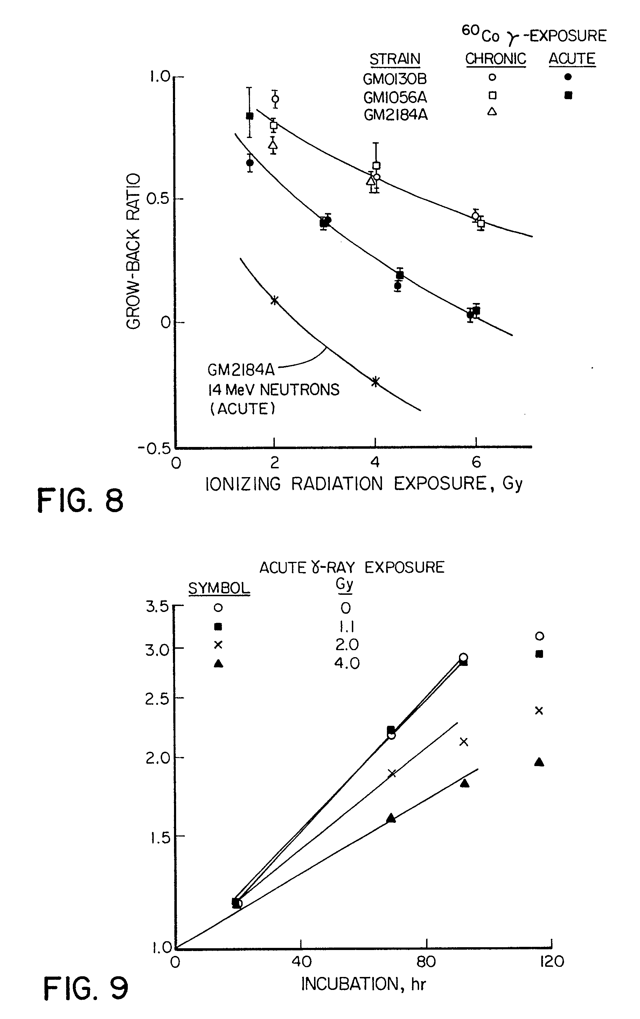

The normal strains (see Fig. 8) show a dose-modifying factor ≃ 2 attributable to dose protraction (i.e. about twice the dose is required in the chronic-γ regime to achieve a given level of effect [i.e. a given GBR value] than is required when acute γ-exposure is employed). This sparing effect on dose protraction is a powerful argument that our response parameter (i.e. GBR) does reflect the ability of irradiated cells to repair/recover from ionizing radiation-induced damage. This ratio closely approaches the dose-rate ratio ($D_{10}$, chronic ÷ $D_{10}$, acute) observed with cultured dermal fibroblasts from normal donors in the colony-forming ability assay (Paterson et al. 1985c).

The A-T heterozygote strain (GM 3382) is sensitive, compared to the normal response, on chronic (Figure 7) but not on acute exposure (Figure 6).

Strains, such as GM1310 and GM 1954, appear to be sensitive on both acute and chronic γ-exposure, and to apparently the same extent relative to normal. This indicates that radiosensitivity in these strains has a different basis from that manifest in A-T-heterozygotes. Consistent with this is a dose-modifying factor of 2 for chronic versus acute γ-ray doses required to achieve equal effect, the same as for the normal strains (Fig. 8). For the GM 1310/GM 1954-type response, both the acute and chronic γ-exposure curves show a steeper dose-response than for normal strains; it may be advantageous to employ somewhat higher doses in their detection (i.e. > 4 Gy).

A preliminary comparison of the effects of low vs. high LET ionizing radiation is possible. Our procedure does demonstrate the greater effectiveness of high LET radiation in killing. The dose-effect curves (grow-back ratio vs. ionizing radiation exposure) for representative normal strains on acute-γ, chronic-γ, and acute exposure to 14 MeV neutrons are shown in Figure 8. The relative biological effectiveness (ratio of the dose of a reference radiation, taken here as acute γ-rays, and of the test radiation required to achieve the same level of effect) is about $2^{1}/_{2}$ for the cytotoxic effect of 14 MeV neutrons.

Validation of the grow-back assay

In summary, we have not only demonstrated the statistical reproducibility of "grow-back" determinations and identified donor radio-sensitivity in A-T compared to normal lymphoblast strains, but we have additionally validated the suitability of our response parameter (the grow-back ratio) in a number of other ways:

    1) Demonstration of a sparing effect of dose protraction,

    2) Demonstration of increased radio-sensitivity using "moderately radiosensitive" strains such as:

        (a) a Roberts syndrome patient (Figure 4; Table I).

        (b) A-T heterozygote strains on chronic $\gamma$-exposure (Figure 4 and Table I) but not acute dose delivery (Figure 6). This is an identical response to that observed using dermal fibroblast strains (Paterson et al. 1985c).

        (c) strain GM 1310, sensitive to both acute-$\gamma$ (Figure 6) and chronic-$\gamma$ (Figure 7). Fibroblasts from this donor have been demonstrated to exhibit abnormal sensitivity to acute-$\gamma$.(see Myers and Gentner, op. cit).

    3) Demonstration of the greater relative biological effectiveness of high LET radiation in killing (Figure 8).

    4) The steeper dose-response of A-T compared to normal strains on acute exposure.


Need for a large normal control series

The need for a large normal control series in an in vitro screening assay has been repeatedly emphasized by many workers because of inherent survival variability. The relatively small control series utilized in this development phase of the grow-back assay was not intended to support (for example) an unambiguous attribution of increased radio-sensitivity to GM 1954, which, out of a repertoire of six apparently normal strains examined in this development phase, appears to be radio-sensitive, both by acute-$\gamma$ test (Figure 6) and by chronic exposure (Figure 7). Only with a large sample of normals in hand will it be possible to assign statistical significance properly.

The need for multiple replicate experiments with each cell line under study should also be emphasized.

The inclusion of the acute exposure test is recommended because it would allow us to differentiate whether a radio sensitive chronic -$\gamma$ response mirrored an A-T heterozygote-type response or was indicative of some other genetic abnormality.


Example 5 Determination of the proportion of persons in the population-at-large who exhibit abnormal sensitivity to ionizing radiation

A major problem in defining hypersensitivity is that the data base on what constitutes a normal response is surprisingly limited most post-radiation survival analyses are done on strains associated with some genetic disorder. We report here our determination, using double-blind conditions and uniform procedures, of the distribution of radioresponse in 270 different strains from ostensibly normal individual donors. We employed both acute and chronic $\gamma$-ray exposure protocols in our screening. Preliminary data from a similar study on a cancer patient population are also given. Our results suggest that an appreciable proportion of normal donors exhibit abnormal radiosensitivity when tested in vitro, and that this characteristic appears to be indicative of a higher-than-normal probability of developing cancer.

270 Lymphoblastoid cell line strains derived from ostensibly normal donors were comprised as follows: 194 were developed from blood samples voluntarily donated by different employees ("Atomic Radiation Workers") at the Chalk River Nuclear Laboratories of Atomic Energy of Canada Limited; 21 (GM strains), derived from "apparently normal" donors, were purchased from the Human Genetic Mutant Cell Repository (HGMCR), Camden, New Jersey; and 55 were developed from Saudi Arabian driver's license applicants by our colleagues B.P. Smith and M.A. Hannan at the King Faisal Specialist Hospital and Research Centre, Riyadh. The basic procedures used to develop lymphoblastoid cell lines have been described (Glick, op., cit.). Eleven strains from persons with A-T (homozygotes) were purchased from HGMCR. Cancer patent strains that we established were derived from blood samples furnished by our colleagues at the Ottawa and London (Ontario) Regional Cancer Clinics.

The cells were cultured and monitored as described above.


Irradiation of cells.

Two dose delivery regimes for $^{60}$Co $\gamma$-irradiation were employed. The "chronic $\gamma$-exposure test" employed a total dose of 4 Gy delivered at 37°C at a dose rate of 0.003 Gy·min$^{-1}$ in a Gamma-Cell 150 beam-port irradiator (Atomic Energy of Canada Limited). The "acute $\gamma$-exposure test" employed a total dose of 1.7 Gy delivered at a dose rate of 2 Gy·min$^{-1}$ in a Gamma-Cell 200 (Atomic Energy of Canada Limited).

Grow-back assay procedures were applied and briefly, the re-growth curves for unirradiated (control) and irradiated cells were established using a Coulter Particle Counter (Coulter Electronics). The ratio of the slopes of the exponential portions ($\Delta\log\cdot h^{-1}$) of the irradiated to the control re-growth curves furnish a "grow-back ratio" (GBR), which was used as our estimator of radioresponse. Use of this ratio corrected for intrinsic differences in growth rate between different strains. The GBR's presented here are preliminary, and represent the unweighted, arithmetic means of all determinations performed on each strain, for a given dose delivery method; more detailed analyses are underway.

11

Screening a "normal" population for abnormal radioresponse

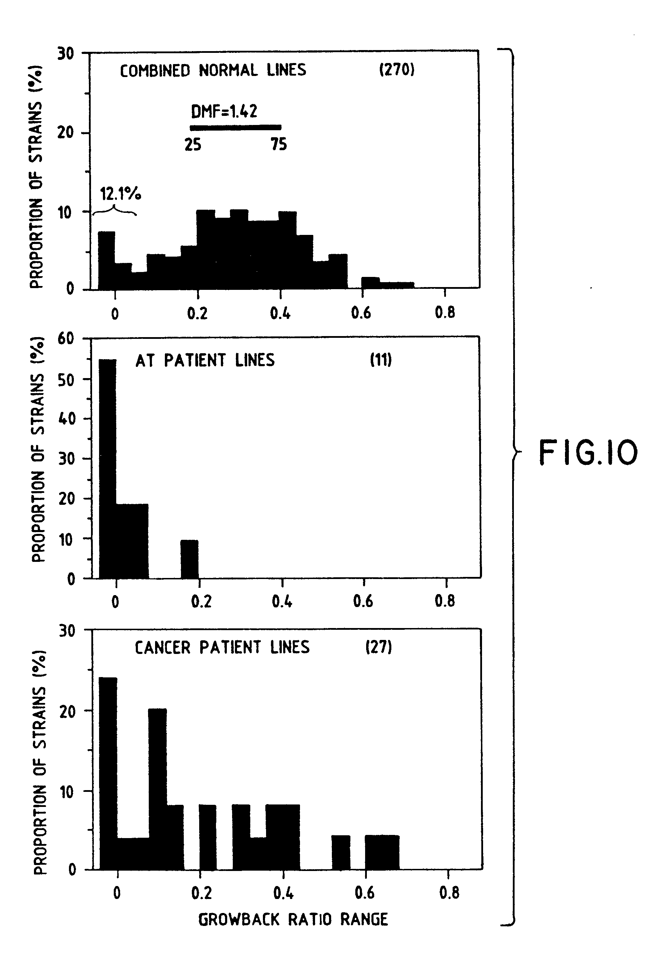

The distribution of radioresponse (percentage of total responses contained within successive 0.04 increments of GBR) is depicted in Figure 10 (upper panel) for the 270 lymphoblastoid cell lines derived from different ostensibly normal donors and assayed "blind" using the chronic γ-irradiation procedure. The inset bar in this panel represents the 25th- to 75th-percentile of responses; it encompasses a range equivalent to a dose-modifying factor $\simeq 1.4$ (this value is derived from curves for GBR versus dose for normal strains. A distinct sub-set of strains, representing some 12% of the total number assayed, is evident at the left side of this distribution. These fall within the distribution of radioresponses for eleven A-T strains (Figure 10, middle panel) that were assayed blind along with the normal strains. An additional 7-8% of strains, representing GBR's of about 0.08-0.18, may be of lesser but still appreciable radiosensitivity. The majority of responses ($\sim$ 80%) are spread over a broad range above a GBR of 0.18.

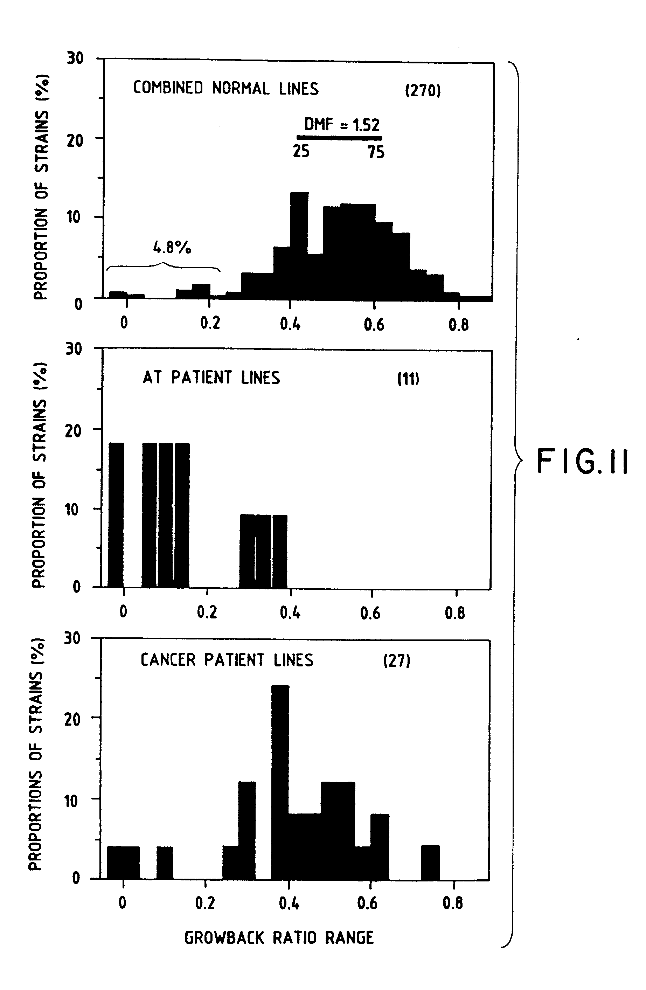

The upper and middle panels of Figure 11 represent the acute γ-irradiation test; otherwise they correspond to Figure 10. Here the distinctly hypersensitive subgroup appears to comprise some 5% of total responses. All strains in this sub-set were contained in the hypersensitive set of responses using the chronic exposure test.

Screening a cancer patient population for abnormal radioresponse

We have embarked on a similar study of the radioresponses of strains derived from freshly-presented, non-selected cancer patients. Of the strains established to date, 27 have been assayed. The results are presented in the lower panels of Figure 10 (for the chronic exposure test) and Figure 11 (for the acute exposure test).

In the chronic exposure test, the proportion of hypersensitive responses is notably greater in the cancer patient strains compared to the ostensibly normal population (compare lower to upper panel, Figure 10). Taking the 25th-percentile point (GBR $\leq$ 0.18) of the ostensibly normal distribution as a division point between "radiosensitive" and "more radioresistant" responses, the difference between the two distributions is highly significant ($p < 0.05$, $\tau^2$ test; Armitage, P, Statistical Methods in Medical Research, New York, Wiley, 1971).

The present results suggest that persons whose lymphoblastoid cells indicate intrinsic hypersensitivity may be at some 4-fold increased risk of developing neoplasia than the remainder. This can be derived, using GBR = 0.18 as an example of an appropriate division point, as follows:

a) For hypersensitive responses, the ratio of cancer patient to "normal" responses is 2.3 (15/27 cancer patients compared to 64/270 normal responses);

b) For more resistant responses (i.e. GBR greater than 0.18), the ratio of cancer patient to "normal" response is 0.58 (12/27 cancer patients versus 206/270 responses in the population of ostensibly normal donors);

c) Therefore the relative risk of cancer in donors whose cultured cells exhibit a hypersensitive response is 4-fold greater (2.3 ÷ 0.58 = 4) than in the remainder of donors whose cultured cells exhibit greater radioresistance.

The plot of GBR's versus donor age for the ostensibly normal population (not shown) furnishes no indication for any change in the proportion of abnormally radiosensitive responses with increasing donor age. This distribution spans the range of ages for cancer patient donors.

The acute γ-irradiation test also provides some indication of a greater proportion in the cancer patient compared to the normal population of highly radiosensitive or "low normal" responses (Figure 11).

The application of this relatively rapid and inexpensive screening assay to a large population of strains from ostensibly normal persons indicates that a substantial proportion form a distinct sub-set of highly radiosensitive responses, as is clear by comparison with the range of response for eleven strains associated with homozygosity for the model cancer-prone, radiosensitive disorder A-T. If the full range of A-T radioresponses (middle panels of Figures 10 and 11) is taken to define radiation hypersensitivity, some one-quarter of the responses of strains from normal donors are included in both tests. These numbers are not out-of-line with results from epidemiological studies. In a U.S. study involving some two thousand consecutively ascertained cancer probands, some 6% were compatible with hereditary cancer and an additional 18% showed familial clustering (using as criterion the existence of two or more first-degree relatives affected with cancer at the same site).

The relatively high proportion, at least 5% (acute test) to 12% (chronic test), of highly radiosensitive (AT homozygote-level) responses is especially remarkable, since A-T occurs at a frequency of only 0.0003-0.0011% of live-births. It may be that the pleiotropic effects which aid identification of A-T have little or nothing to do with ionizing radiation hypersensitivity per se. These observed hypersensitive responses also seem unlikely to include A-T carriers; all A-T heterozygote radioresponses we have obtained thus far fall above the 25th-percentile of the range of normals.

Further, hypersensitive responses in vitro appear to indicate a greater-than-normal probability of developing cancer: these responses are significantly enriched in a population of strains from cancer patients compared to the control population. Whether environmental exposure to ionizing radiation or to radiomimetic agents may be responsible for this apparent cancer proneness is not known. It would, however, seem prudent to assume that the relative risk for radiogenic cancer in such donors may be at least as great as for spontaneous cancer. Overall, donors whose cells exhibit hypersensitivity in vitro appear to have a 4-fold greater risk of developing cancer than does the remaining majority ($\sim$ 75%). If this latter group, which itself spans an appreciable range of response, is shown to include strains of intermediate radiosensitivity and to be associated with intermediate risk of neoplasia, the risk ratio may be even higher.

We conclude from Figures 10 and 11 that a chronic exposure testing regime may have greater utility in defining hypersensitive responses, and the excess risk of neoplasia that may be associated with such responses, than does an acute exposure test of the sort employed nearly universally.

One of the cancer patients in the class of greatest radiosensitivity in both the chronic and acute exposure tests was a woman with rectal cancer who exhibited a severe bowel reaction to radiotherapy after only 26 Gy and had to have treatment stopped; thus our assay may well serve as a prognostic indicator for adverse radioreaction. Perhaps more importantly, this prematurely terminated treatment turned out to be highly efficient in tumour control, resulting in one of the most rapid regressions ever seen at the Ottawa Cancer Clinic for this tumour type. Our radioresponse measurement suggests that, in fact, this patient received a highly aggressive exposure relative to the ability of her cells to handle such damage, indicating that 'tailor-made' treatment rigour predicated on individual radiosensitivity may allow the development of more effective tumour management protocols. Given the considerable variation extant in the population it would seem that no one level of treatment is appropriate for all patients subjected to radiotherapy. Our data support the suggestion that radiotherapy treatment rigour at present may be biased by hypersensitive responses. It would follow that tumour treatment in more radioresistant persons may be suboptimal. There exists evidence for head and neck cancer patients, for example, that radiation resistance and repair proficiency in their tumour cells is associated with unsuccessful radiotherapy outcomes; we suggest that these different tumour cell responses may largely reflect different intrinsic radiosensitivities of the patients (cf. Figure 10, lower panel) as measured by assays such as ours. Thus while the immediate goal of this project is to indicate where prevention of cancer, if possible, may be achieved, application of this assay may support improved strategies for cancer treatment as well.

We have recently obtained another highly radiosensitive cancer patient response in vitro. This person was not included in the non-selected cancer patient results in Figures 10 or 11. This donor was a breast cancer patient who had a marked reaction to radiotherapy after only 20 Gy in eight fractions. The concordance in these two cases between hypersensitivity in vitro and marked radioreaction in vivo strongly indicates that a low GBR in our screening assay does indeed reflect increased radiosensitivity in the donor.

In radiation protection and in occupational health protection generally, control of the risk associated with potential exposure of humans is achieved by controlling exposure. The data presented here indicate that, for radiation protection purposes at least, exposure may not be an entirely appropriate surrogate for risk. The concept of "equal exposure -- equal risk" assumes a homogeneous response among individuals which seems not to be tenable. Armed with this knowledge, however, and employing this or a similar screening assay, some novel strategies for achieving improved protection become apparent. These include: (a) to vary individual allowable exposure limits to place everybody at approximately equivalent risk (that is, to protect everybody to the same extent); (b) to base protection on the response of the more sensitive/susceptible persons in the population; (c) to minimize the potential exposure of those who may be more susceptible. This latter route would seem likely to result in a net gain in radiation protection even employing present exposure limits, and may be the most prudent immediate course of action.

### Example 6 The effect of a chemical, mitomycin C, on grow-back

Related work on the range of sensitivity found in 76 strains of apparently normal individuals to the DNA-crosslinking agent mitomycin C was performed. The exposure regime (outlined above) represents a "chronic" exposure that we have developed for this agent; notably less resolution in detecting sensitivity in model strains was seen when a conventional acute (1 hr treatment) exposure regime was employed. A very broad distribution of sensitivities was evident. The position of model cancer-prone strains (from patients with Fanconi anemia and Bloom syndrome, the former clearly related to hypersensitivity to agents causing DNA double-strand damage) or a plot of proportion of cell lines against slope ratio range was clearly at the low end of the slope ratio range. A parallel set of results was obtained using 33 lines derived from donors who have or had cancer. While the numbers studied to date are small, the distribution of slope ratios appears to be shifted towards a greater degree of sensitivity (the low end of the slope ratio range).

### Example 7 The effect of acute gamma irradiation on interleukin-2 stimulated lymphocytes

Interleukin-2 stimulated lymphocytes were subjected to 0, 1.1, 2 or 4 Gy of gamma radiation at an acute rate. Figure 9 shows the results of such irradiation on the stimulated lymphocytes. At an acute dose of 2 Gy or more there is a clear effect shown on grow-back response which is even more clearly shown at the higher 4 Gy dose.

Peripheral blood lymphocytes have been found particularly useful in the grow-back assay. The cells are easy to obtain and the test yields results very quickly. This means that, in addition to being less expensive, the test is also of more immediate benefit to a patient.

### Proposed screening assay

An approach to using the grow-back assay to assess the susceptibility of an individual to a possibly toxic agent comprises:

    (a) taking a cell sample from the individual, e.g., peripheral blood lymphocytes;

    (b) performing grow-back responses (after treatment and with a control) on subsamples of the cell sample; and

(c) comparing the grow-back responses of the cell sample from the individual under test with a library of grow-back responses to the same treatment obtained for a plurality of reference cell strains to assess if the individual is abnormally susceptible or resistant or shows a "normal" response.

This approach should be particularly useful in treating cancer patients with radiation or chemicals, or both. The dose could now be tailored to maximize the curative treatment while minimizing the side effects. Hitherto doses have been determined on an empirical and historical basis of what was found to be effective on patients in general, only accounting for a particular patient's susceptibility or ability to take a higher than normal dose by trial and error on the patient rather than on cell samples taken from the patient.

Cell samples for candidates for cancer treatment could be subjected to the methods of the invention and the results obtained used to recommend a treatment regime tailored to the patient. The information the treating physician needs is whether or not the patient is abnormally susceptible to the proposed treatment; if so, then the dose should be correspondingly reduced. Of course, if the patient appears to be abnormally resistant to the proposed treatment then an increased dose can be used.

The Utility of the Grow-Back Method in Cancer Therapy

Inherent differences in the responses of individuals to the radiation or chemical insult delivered in therapy, as measured by in vitro assays, should be reflected by in vivo responses of their cancer tissue or cells to the treatment regime. Varying response to radiotherapy and chemotherapy among different patients is commonly inferred clinically at present. Although doses are not now planned with sensitivity under consideration, occasionally the dosage is crudely adjusted based upon patient's response to treatment. The use of an assay, such as the grow-back assay, to gauge an individual's sensitivity to the therapeutic agent before treatment, will result in improved treatment regimes. An early example of this recently occurred in the successful treatment of an A-T homozygote with radiotherapy at a reduced dose. A-T homozygotes exhibit extreme sensitivity in our radiosensitivity assay and are known to face a risk of cancer several hundred fold that of an age-matched control population.

Other Utilities

The grow-back method has potential for use as a means for occupational screening of workers at risk (a) to see if they are sensitive to any genotoxic agent to which they are exposed in the course of their employment and (b) to subject job applicants to predisposition screening for insurance and other purposes.

One benefit of the method is that the response of an individual, as measured by treatments and responses of cells derived from that individual, can be assayed. Dose modifying factors, if present, can be detected. For example an individual with an unusually efficient cell repair system for the damage induced by a particular genotoxic agent may have graphed survival slopes of control and treated samples of cells which differ little, if at all at a chronic dose when compared with other individuals of the population but shows graphed survival slopes which are not out of the ordinary at acute doses of the same agent. Conversely, an individual with a defective cell repair system may be distinguished by the response of cells at a chronic dose (and not be so distinguished by the response of cells at an acute dose). Of course, the above presupposes and requires that a sufficient number of individuals have been screened to provide a measure of mean and variance of the response of the base population.

Under most circumstances (disasters excepted) individuals at risk are exposed to chronic rather than acute doses of genotoxic agents. Therefore, the ability of this test to discrim- inate those among the population that are particularly susceptible to particular genotoxic agents is especially valuable to those industries where such genotoxic agents comprise an occupational hazard.

The grow-back assay, in conjunction with a set of reference cell strains which includes strains with a range of susceptibilities to known toxic agents, can be used to screen previously untested or new and potentially toxic agents or combinations of such agents. In this way, previously unknown environmental hazards can be highlighted and such possible hazards avoided.

In addition to the above, the grow-back assay is of potential scientific and technical interest in investigating the mechanism underlying genetic disorders. It also can be used to investigate cell repair mechanisms in any organism from which cell samples can be taken, including single-celled organisms such as yeasts.

14

## TABLE I

"GROWBACK ASSAY" FOR SEVERAL LYMPHOBLASTOID CELL LINES FOLLOWING 4 Gy CHRONIC EXPOSURE TO $^{60}$Co $\gamma$-RAYS.

These parameters are derived from the data depicted in Figs.2, 3 and 4.

| Strain | Description | Slope of exponential portion[1] ,hr$^{-1}$ | | Growback ratio[2] |
|--------|-------------|--------------|--------|-------------------|
| | | unirradiated | 4 Gy | |
| GM2184A | Normal | 0.01403 | 0.00842 | 0.600 |
| GM1056A | Normal | 0.01446 | 0.00786 | 0.544 |
| GM3189 | A-T homozygote | 0.01106 | -0.00126 | -0.114 |
| GM2782 | A-T homozygote | 0.00722 | -0.00110 | -0.152 |
| GM3382 | A-T heterozygote | 0.01194 | 0.00362 | 0.303 |
| R20 | Roberts syndrome | 0.01019 | 0.00146 | 0.143 |

(1) $\Delta\log_{10}(N_t/N_o) \div \Delta t$, using the exponential portion of the growth or "growback" curve; units are hr$^{-1}$.
(2) Slope of the exponential portion of the growback response for the irradiated population divided by that for the unirradiated (control) population for that strain, as discussed.

## Claims

1. A method for determining the susceptibility of an organism to potentially toxic agent which comprises:
   (i) providing a control sample and a treatment sample from cells derived from the organism;
   (ii) contacting the treatment sample with the potentially toxic agent;
   (iii) incubating the cells of the treatment and control samples, after step (ii), in nutrient medium and then assaying the apparent number of intact cells; and
   (iv) determining a measure of susceptibility of the organism to the potentially toxic agent from a comparison of the change in apparent number of intact cells in the control and treatment samples over time.

2. A method as claimed in claim 1 characterised in that the potentially toxic agent is a genotoxic agent or an agent which can interfere with cell growth or viability.

3. A method as claimed in claim 1 or claim 2 characterised in that the potentially toxic agent is a form of ionizing radiation or a chemical capable of alterning DNA.

4. A method as claimed in claim 3 characterised in that the chemical is an alkylating agent.

5. A method as claimed in any of claims 1 to 4 characterised in that the measure of susceptibility is represented by graphed survival slopes of control and treatment samples reflecting relative rates of grow-back of dividing cells.

6. A method as claimed in any one of claims 1 to 5 characterised in that the measure of susceptibility is represented by graphed survival slopes of control and treatment samples reflecting relative death rates of unstimulated lymphocytes.

7. A method as claimed in any one of claims 1 to 6 characterised in that the method additionally comprises the determining or diagnosing of the relative susceptibility of the organism to the potentially toxic agent compared with a reference population of other individual organisms of the same species by
   (v) comparing the measure of susceptibility of the organism to measures of susceptibility of other individual organisms of the same species in the reference population thereby obtaining a measure of relative susceptibility of the organism to the agent.

8. A kit for measuring susceptibility of a test subject to a potentially toxic agent which kit comprises at least first and second components which first and second components are in separate containers, the first component comprising at least one reference cell line, each cell line being a separate container, and the second component comprising at least one incubating medium for grow-back of control and treated samples of each cell line.

9. A multicomponent kit for determining the range and extent in susceptibility of individuals in a population of a species of organism to a potentially toxic agent, which kit comprises a plurality of reference cell strains as components, each component being in a separate container, the reference cell strains having previously been tested for susceptibility to the at least one toxic agent, the reference cells being selected to vary in susceptibility to at least one toxic agent among the reference cell strains in a manner which relects a range of susceptibility of representative individuals sampled from the population of the organism, and the plurality of reference cell strains being large enough to be representative of the range of variation expected in the population.

10. A method for determining a therapeutic dose or determining or monitoring a treatment regime of an anti-neoplastic agent to be administered to an individual organism suspected of having a neoplasm which method comprises:

(i) providing a control sample and a treatment sample from cells derived from the individual organism;

(ii) contacting the treatment sample with the anti-neoplastic agent;

(iii) incubating the cells of the treatment and control samples, after step (ii), in nutrient medium and then assaying the apparent number of intact cells;

(iv) determining a measure of susceptibility of the individual organism to the anti-neoplastic agent from a comparison of the change in apparent number of intact cells in the control and treatment samples over time; and

(v) from the measure of susceptibility, determining a therapeutic dose or determining or monitoring a treatment regime of the anti-neoplastic agent for the individual organism and any toxic side effects of the anti-neoplastic agent on the individual organism.

11. A method as claimed in claim 10 wherein the therapeutic dose for the individual organism is about the dose which produces the largest difference between a survival rate of neoplasm treatment and a mortality rate from any toxic side effects of the anti-neoplastic agent.

FIG. 1

FIG. 2

FIG. 3

FIG. 4

FIG. 5

FIG. 7

FIG. 6

FIG. 8

FIG. 9

FIG.10

FIG.11